(19) Europäisches Patentamt
European Patent Office
Office européen des brevets

(11) Veröffentlichungsnummer: **0 047 945 B1**

(12) # EUROPÄISCHE PATENTSCHRIFT

(45) Veröffentlichungstag der Patentschrift: **21.09.83**

(51) Int. Cl.³: **C 07 C 143/70,** C 07 C 139/00, C 25 B 3/02

(21) Anmeldenummer: **81106997.0**

(22) Anmeldetag: **07.09.81**

(54) **Verfahren zur Herstellung von Perfluorcarbonylsulfonsäurefluoriden sowie die dabei als Zwischenprodukte auftretenden Fluorsulfatoperfluoralkansulfonsäurehalogenide.**

(30) Priorität: **12.09.80 DE 3034550**

(43) Veröffentlichungstag der Anmeldung: **24.03.82 Patentblatt 82/12**

(45) Bekanntmachung des Hinweises auf die Patenterteilung: **21.09.83 Patentblatt 83/38**

(84) Benannte Vertragsstaaten: **BE CH DE FR GB IT LI NL**

(56) Entgegenhaltungen:
**DE-A-1 912 738**
**DE-A-2 234 837**
**DE-A-2 725 211**

(73) Patentinhaber: **HOECHST AKTIENGESELLSCHAFT, Postfach 80 03 20, D-6230 Frankfurt am Main 80 (DE)**

(72) Erfinder: **Millauer, Hans, Dr., An den sieben Bäumen 21b, D-6236 Eschborn (DE)**
Erfinder: **Schwertfeger, Werner, Dr., Erlenstrasse 8, D-6306 Langgöns (DE)**

EP 0 047 945 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von Perfluorcarbonylsulfonsäurefluoriden

Verfahren zur Herstellung von Perfluorcarbonylsulfonsäurefluoriden sowie die dabei als Zwischenprodukte auftretenden Fluorsulfatoperfluoralkansulfonsäurehalogenide.

Perfluorcarbonylverbindungen sind hauptsächlich Zwischenprodukte in der organischen Fluorchemie.

Sie lassen sich z.B. mit Hexafluorpropenepoxid zu Perfluorcarbonsäurefluoriden umsetzen, die bei der Pyrolyse perfluorierte Vinylverbindungen liefern:

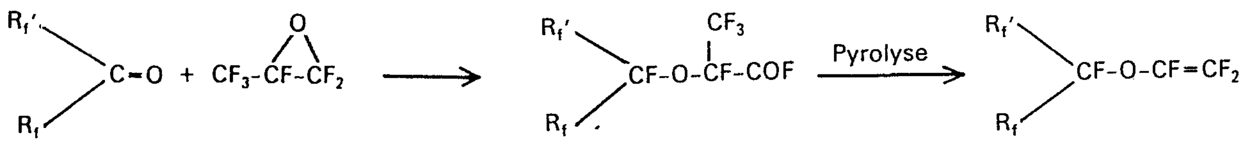


$R_f$ = Perfluoralkyl, $R_f'$ = Fluor oder Perfluoralkyl.

Auch perfluorierte Allylether sind aus Perfluorcarbonylverbindungen herstellbar (DE-OS 2 753 886):

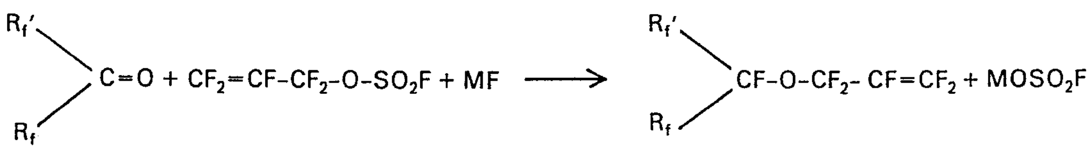

$R_f$ = Perfluoralkyl, $R_f'$ = Fluor oder Perfluoralkyl, M = Alkalimetall.

Die perfluorierten Vinyl- oder Allylether können durch Homo- oder Copolymerisation in wertvolle, chemisch und thermisch ausserordentlich beständige Oligomere und Polymere überführt werden. Die Oligomeren mit flüssiger Konsistenz dienen z.B. als Schmier- und Gleitmittel sowie Hydraulikflüssigkeiten, die Polymeren mit fester Konsistenz u.a. als Überzugsmaterialien, Elastomere, Ionenaustauscher (wenn noch saure oder basische Gruppen vorhanden sind) etc.

Ausgehend von Perfluorcarbonylverbindungen, die noch eine Sulfonsäurefluoridgruppe enthalten, wurden auch bereits perfluorierte Vinyl- oder Allylether mit einer Sulfonsäurefluoridgruppe hergestellt, die nach der Copolymerisation mit Tetrafluorethylen und anschliessender Hydrolyse der $-SO_2-F$-Gruppe ein Kationenaustauscherharz lieferten.

So geht z.B. das in US-PS 3 301 893 und US-PS 3 282 875 beschriebene Verfahren von Fluorsulfonyldifluoressigsäurefluorid aus. Die Reaktion mit Hexafluorpropenepoxid und nachfolgende Pyrolyse ergibt einen perfluorierten Vinylether mit einer Sulfonsäurefluoridgruppe:

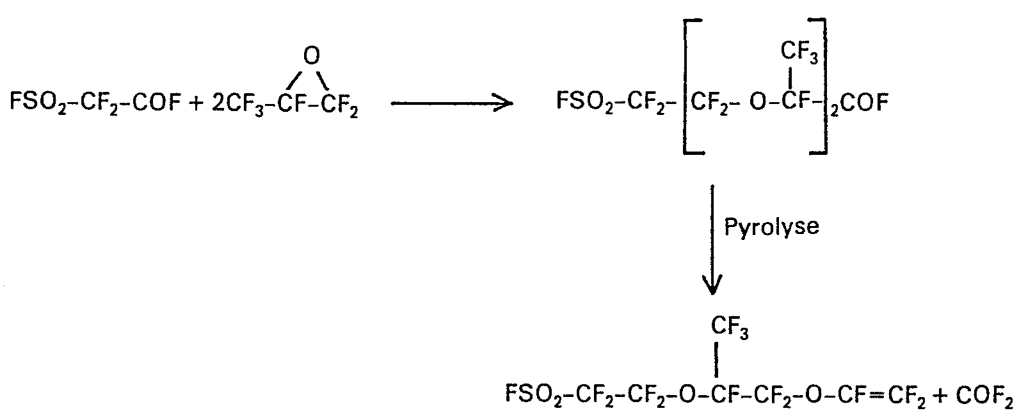


Der Vinylether wird mit Tetrafluorethylen copolymerisiert. Die Hydrolyse des Copolymerisates ergibt dann das Kationenaustauscherharz.

In der DE-OS 2 753 886 ist die Reaktion von 2-Oxopentafluorpropansulfonsäure-1.1-difluorethylester mit Kaliumfluorid und Perfluorallylfluorosulfat beschrieben:

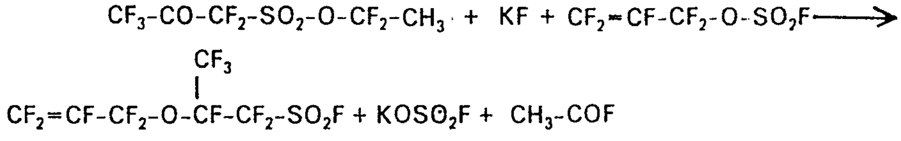

Als Zwischenprodukt tritt bei dieser Reaktion 2-Oxopentafluorpropansulfonsäurefluorid ($CF_3$-CO-$CF_2SO_2F$) auf.

Der erhaltene perfluorierte Allylether mit einer Sulfonsäurefluoridgruppe wird mit Tetrafluorethylen copolymerisiert und das erhaltene Copolymerisat zu einem Ionenaustauscherharz hydrolysiert.

Die bei diesen Verfahren als Ausgangsmaterialien verwendeten perfluorierten Carbonylverbindungen mit eienr Sulfonsäurefluoridgruppe können nach verschiedenen Verfahren hergestellt werden.

Fluorsulfonyldifluoressigsäurefluorid wird z.B. durch Umsetzung von Tetrafluorethylen mit Schwefeltrioxid und Umlagerung des erhaltenen Sultons mit Triethylamin erhalten [J. Am. Chem. Soc. 82, 6181 (1960)]:

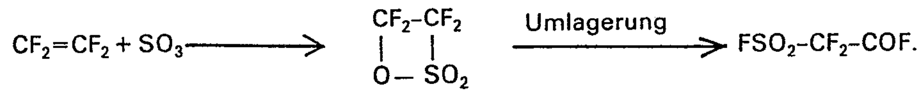


Die Herstellung des Sultons ist jedoch mit Explosionsgefahr verbunden, weil Schwefeltrioxid mit bereits gebildetem Sulton eine exotherme Reaktion ergeben kann [Chem. Eng. News 49, Heft 22, S. 3 (1971)]:

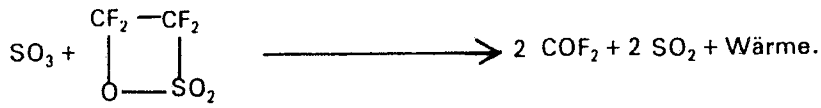

2-Oxopentafluorpropansulfonsäurefluorid ist bisher nicht isoliert worden. Es wurde jedoch beschrieben, dass die Verbindung bei der Umsetzung von 2-Oxopentafluorpropansulfonsäure-1.1-difluorethylester mit Kaliumfluorid entsteht (DE-OS 2 753 886):

$$CF_3\text{-}CO\text{-}CF_2\text{-}SO_2\text{-}O\text{-}CF_2\text{-}CH_3 \xrightarrow{KF} [CF_3\text{-}CO\text{-}CF_2\text{-}SO_2F] + FOC\text{-}CH_3.$$

2-Oxopentafluorpropansulfonsäure-1.1-difluorethylester, der sich in Glasgefässen zersetzt und deshalb in Polyethylenflaschen aufbewahrt werden muss, wird auf folgendem Weg hergestellt:

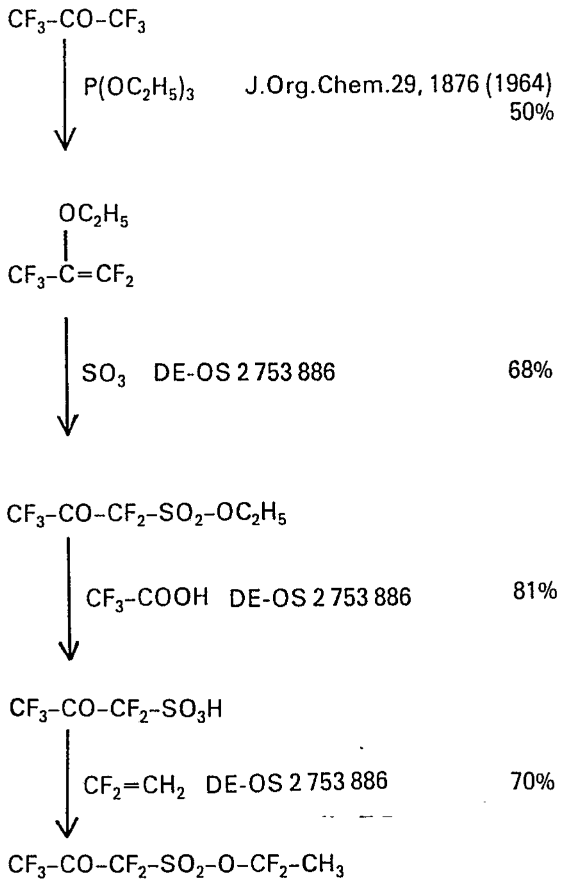


Aufgabe dieser Erfindung war es nun, einen gefahrlosen allgemein anwendbaren und einfachen Weg zur Herstellung von Perfluorcarbonylsulfonsäurefluoriden zu finden.

Erfindungsgegenstand ist ein Verfahren zur Herstellung von Perfluorcarbonyl-sulfonsäurefluoriden der Formel I:

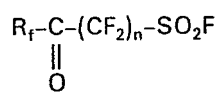 (I),

worin $R_f$ = F oder
Perfluoralkyl mit 1–10, vorzugsweise 1–8, insbesondere 1–3 C-Atomen und
n = 0–7, bedeuten,
das dadurch gekennzeichnet ist, dass man

a) Monohydroperfluoralkansulfonsäurehalogenide der Formel II

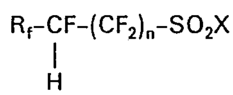 (II),

worin $R_f$ und n die gleiche Bedeutung wie in Formel I besitzen und X Chlor oder Fluor, vorzugsweise Fluor bedeutet, in einem Elektrolyten aus Fluorsulfonsäure und einem Alkalifluorsulfonat unter Verwendung von Anoden aus glasartigem Kohlenstoff
und von Kathoden aus einem üblichen, jedoch unter den Elektrolysebedingungen stabilen Material elektrolysiert, und

b) die dabei erhaltenen Fluorsulfatoperfluoralkansulfonsäurehalogenide der Formel III

$$R_f\text{-}\underset{\displaystyle |\atop \displaystyle OSO_2F}{CF}\text{-}(CF_2)_n\text{-}SO_2X \qquad (III),$$

worin $R_f$, X und n die gleiche Bedeutung wie in den Formeln I und II besitzen, in Gegenwart von mindestens einem Alkalifluorid zu den Perfluorcarbonylsulfonsäurefluoriden der Formel I umsetzt.

Die für das erfindungsgemässe Verfahren benötigten Ausgangsverbindungen der Formel II (mit n = 1-7) können nach dem Verfahren der gleichzeitig eingereichten und auch prioritätsgleichen EP-A-47 946 auf folgendem Weg erhalten werden:

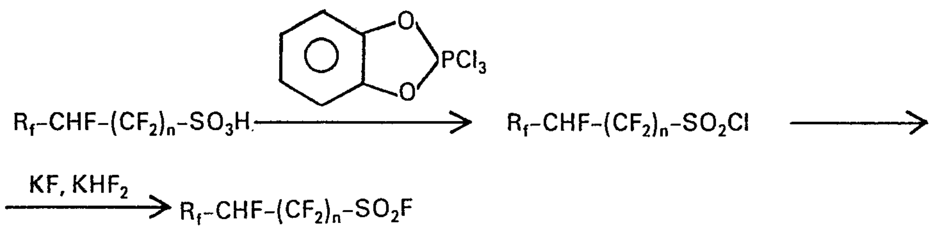


$R_f$ = Fluor oder Perfluoralkyl, n = 1 bis 7.

Die Ausgangsverbindungen mit n = 0 sind z.B. in folgender Literatur beschrieben bzw. nach den darin beschriebenen Verfahren erhältlich:

$CF_3$-CHF-$SO_2F$ (Herst. nach Izv. Akad. Nauk, SSSR Ser. Khim. 1967, 1685, englische Übersetzung)

$CHF_2$-$SO_2F$

[Herst. von $CHF_2$-$SO_2Cl$ nach J. Org. Chem. 44, 1708 (1979)] und daraus das $CHF_2$-$SO_2F$ nach dem Verfahren der vorerwähnten EP-A-47 946.

Stufe a) des Verfahrens:

Die Herstellung der Fluorsulfatoperfluoralkansulfonsäurehalogenide, vorzugsweise der -fluoride, der Formel III erfolgt durch anodische Substitution des Wasserstoffatoms der entsprechenden Monohydroperfluoralkansulfonsäurehalogenide, vorzugsweise der -fluoride, der Formel II.

Derartige Substitutionsreaktionen sind bei polyfluorierten Verbindungen mit einem primären Wasserstoffatom ($-CHF_2$) bereits beschrieben worden. In J. Chem. Soc. Chem. Comm. 1978, 118 ist die anodische «Funktionalisierung» von 1-Hydroperfluoralkanen ohne weitere funktionelle Gruppe in einem Elektrolytsystem aus Fluorsulfonsäure und Kaliumfluorsulfonat als Leitsalz in einer ungeteilten Zelle an Platinelektroden zu 1-Fluorsulfatoperfluoralkanen beschrieben. Die Autoren vermuten als entscheidendes Zwischenprodukt das äusserst reaktionsfähige Peroxodisulfuryldifluorid ($FSO_2$-O-O-$SO_2F$), welches unter den Elektrolysebedingungen an der Anode gebildet wird und ohne Isolierung mit der im Elektrolyten anwesenden Fluorverbindung abreagiert.

Die Umsetzung von Monohydroperfluorverbindungen mit reinem Peroxodisulfuryldifluorid ist ebenfalls bereits beschrieben worden [J. Fluorine Chem. 2, 173 (1972/73)]. Unter diesen Bedingungen ist es sogar möglich, Wasserstoffatome in Molekülen zu ersetzen, die bereits eine funktionelle Gruppe, z.B. eine Nitrilgruppe (Ausbeute 22%), enthalten. Dennoch ist allgemeinen der elektrochemischen Methode der Vorzug zu geben, da in diesem Fall die gefährliche, getrennte Herstellung des Peroxodisulfuryldifluorids aus Fluor und Schwefeltrioxid entfällt. Das bei der elektrochemischen Methode in situ erzeugte Reagenz braucht dagegen nur in geringen, stationären Konzentrationen vorhanden zu sein und kann gefahrloser umgesetzt werden. Allerdings ist die elektrochemische Methode a priori, z.B. wegen Nebenreaktionen mit dem Elektrolyten oder Nebenprozessen an der Kathode, insbesondere bei Verbindungen mit einer weiteren funktionellen Gruppe, nicht generell einsetzbar. So können beispielsweise in Verbindungen mit der oben erwähnten Nitrilgruppe keine Fluorsulfatogruppen durch anodische Substitution eingeführt werden. Im Falle des erfindungsgemässen Verfahrens verläuft die anodische Substitution dennoch befriedigend, obgleich das im Stand der Technik beschriebene Anodenmaterial sich als ungeeignet erwies. Diese Elektroden neigen zu relativ hoher Korrosion, wobei das abgelöste Platin zum Teil als Schlamm und zum Teil in Lösung verbleibt. Besonders nachteilig wirkt sich die teilweise Wiederabscheidung des anodisch gelösten Platins an der Kathode aus, die zu schlecht leitfähigen Deckschichten und zu hohen Zellspannungen führt. Die Lösung dieses Problems beruht auf der Verwendung von glasartigem Kohlenstoff anstelle von Platin als Anodenmaterial. Dieses Material erweist sich als unerwartet korrosionsbeständig, obwohl andere Kohlenstoffelektroden, wie Elektrodengraphite oder imprägnierte Apparategraphite, augenblicklich vom Elektrolyten, sogar ohne Stromfluss zerstört werden.

Glasartiger Kohlenstoff eignet sich neben anderen Stoffen wie beispielsweise Platin oder Edelstahl auch als Kathodenmaterial. Über Herstellung, Struktur und Eigenschaften von glasartigem Kohlenstoff siehe z.B. den Artikel «Thermischer Abbau von Polymeren bis zum elementaren Kohlenstoff – ein Weg zu Werkstoffen der Zukunft» von E. Fitzer in Angew. Chem. 92. (1980) 375-386.

Die Elektrolyse kann in einfachen, ungeteilten Zellen durchgeführt werden. Durch poröse Diaphragmen geteilte Zellen können eine Verbesserung der Stromausbeute bewirken, da sie unerwünschte Nebenreaktionen wie z.B. die kathodische Rückspaltung des Peroxodisulfuryldifluorids unterdrücken. Das Flächenverhältnis von Anode zu Kathode liegt zwischen etwa 1:1 bis vorzugsweise etwa 5:1 bis 10:1.

Zur Bereitung des Grundelektrolyten, welcher erfindungsgemäss aus Fluorsulfonsäure und einem darin gelösten Alkalifluorsulfonat besteht, löst man zweckmässig ein entsprechendes, leicht zugängliches Alkalichlorid, wie beispielsweise Lithiumchlorid, Natriumchlorid oder Kaliumchlorid, in Fluorsulfonsäure, die man gegebenenfalls einer Reinigung durch fraktionierte Destillation unterzogen hat, wobei sofort die Hauptmenge an Chlorwasserstoff aus der Lösung entweicht. Der Rest wird durch Einleiten von trockenem Stickstoff ausgetrieben. Die anzuwendende Konzentration des Alkalisulfonats im Grundelektrolyten ist nicht kritisch und liegt normalerweise im Bereich von etwa 0.05 bis etwa 3 Mol pro Liter. Oxidierbare Verunreinigungen oder Feuchtigkeitsspuren werden gegebenenfalls durch eine Vorelektrolyse beseitigt.

Die als Ausgangsstoffe benutzten Monohydroperfluoralkansulfonsäurehalogenide II werden im Grundelektrolyten gelöst oder dispergiert, wobei Gemische mit bis zu etwa 60% der Ausgangsverbindung, bezogen auf den Grundelektrolyten zur Anwendung kommen können. Die Elektrolyse wird vorteilhaft bei einer anodischen Stromdichte von etwa 10 - 150 $mA.cm^{-2}$, vorzugsweise etwa 20 - 80 $mA.cm^{-2}$, und einer Temperatur von 0 - 100°C, vorzugsweise 20 - 40°C betrieben.

Die Aufarbeitung der Elektrolysegemische und die Isolierung der Fluorsulfatoperfluoralkansulfonsäurehalogenide erfolgt in an sich bekannter Weise. Bei zweiphasigen Reaktionsgemischen ist es zweckmässig, die fluororganische Phase, die gegebenenfalls noch wenig Fluorsulfonsäure enthält, durch Scheiden abzutrennen. Andernfalls muss das Elektrolyseprodukt von dem Grundelektrolyten destillativ abgetrennt werden. In beiden Fällen kann die Elektrolytphase bzw. der Destillationssumpf nach Zugabe frischer Fluorsulfonsäure wieder in die Elektrolysestufe recyclisiert werden.

Die rohren Fluorsulfatoperfluoralkansulfonsäurehalogenide können durch fraktionierte Destillation weiter gereinigt werden.

Sie sind neue Verbindungen.

Stufe b) des Verfahrens:

Erfindungsgemäss werden aus den Fluorsulfatoperfluoralkansulfohalogeniden der Formel III durch Umsetzung mit katalytischen Mengen eines Alkalifluorids (LiF, NaF, KF, RbF, CsF) Perfluorcarbonylverbindungen mit einer Sulfofluoridgruppe der Formel I hergestellt. Für X = Cl in Formel III muss jedoch zusätzlich mindestens die zu III equimolare Menge Alkalifluorid eingesetzt werden.

Die Spaltung von primären und sekundären Fluorsulfaten mit Alkalifluoriden ist bereits mehrfach publiziert worden:

So ist z.B. in Inorg. Chem. 3, 287 (1964) die Zersetzung eines eine primäre und eine sekundäre Fluorsulfatogruppe enthaltenden Moleküls beschrieben worden.

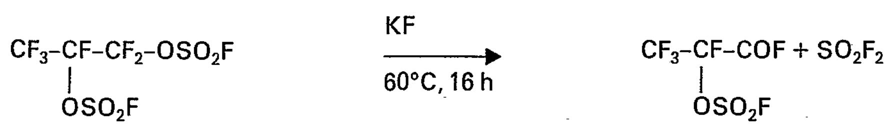


Dabei wird nur die primäre Fluorsulfatogruppe angegriffen. Wird CsF stat KF verwendet, so tritt Zerstörung des gesamten Moleküls ein. Mengenangaben fehlen.

Auch in Inorg. Chem. 4, 1441 (1965) werden primäre und sekundäre Fluorosulfate mit Kaliumfluorid umgesetzt.

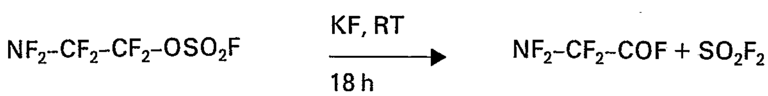


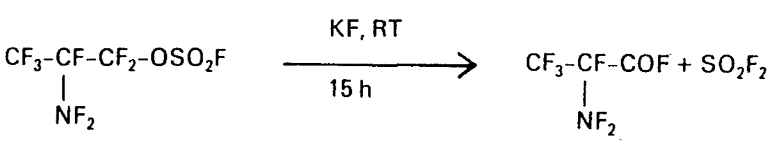


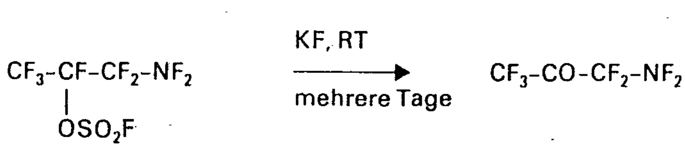


Bei diesen Reaktionen wurde das Kaliumfluorid in einem etwa 50 molaren Überschuss pro Mol Fluorosulfat eingesetzt.

J. Fluorine Chem. 14, 519 (1979) beschreibt die Umsetzung von primären Fluorosulfaten mit Caesiumfluorid

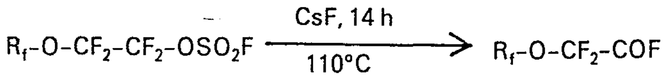


$R_f = CF_3, SF_5$

Bei dieser Reaktion werden das Fluorosulfat und das CsF im Molverhältnis von 1:20 eingesetzt.

Mit einem grossen Überschuss an KF wird die in Inorg. Chem. 18, 3281 (1979) beschriebene Zersetzung eines sekundären Fluorosulfats durchgeführt. Hier tritt zusätzlich noch eine Umlagerung auf:

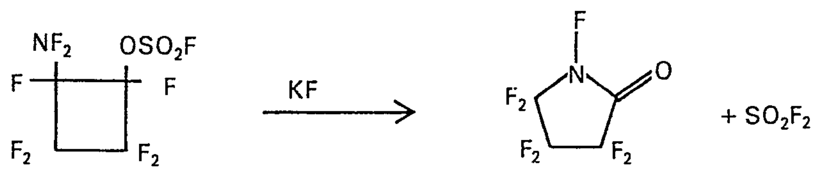


Auch in Inorg. Chem. 5, 2184 (1966) ist die Zersetzung von sekundären Fluorosulfaten beschrieben

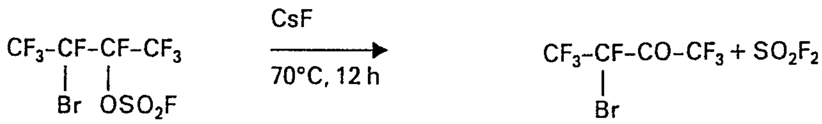


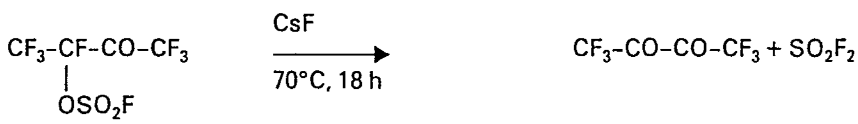


Während im zweiten Fall ein grosser Überschuss an Caesiumfluorid eingesetzt wird, sind im ersten Fall keine Mengenverhältnisse angegeben. Es muss jedoch davon ausgegangen werden, dass wie üblich eine mehr als equimolare Menge des Caesiumfluorides pro Mol Fluorosulfat verwendet wurde.

Allen diesen Reaktionen ist gemeinsam, dass ohne Lösungsmittel gearbeitet wird. In diesen Fällen scheint ein erheblicher Überschuss an Alkalifluorid für das Gelingen der Reaktion erforderlich zu sein.

Es gibt jedoch auch Beispiele dafür, dass für solche Reaktionen katalytische oder equimolare Mengen an Alkalifluorid verwendet werden können, nämlich dann, wenn ein aprotisch polares Lösungsmittel wie Diglyme oder Acetonitril eingesetzt wird.

In Izv. Akad. Nauk SSSR, Ser. Khim. 1973, 2659, (engl. Ausgabe) ist eine solche Reaktion für ein primäres Fluorosulfat (mit katalytischen Mengen an KF):

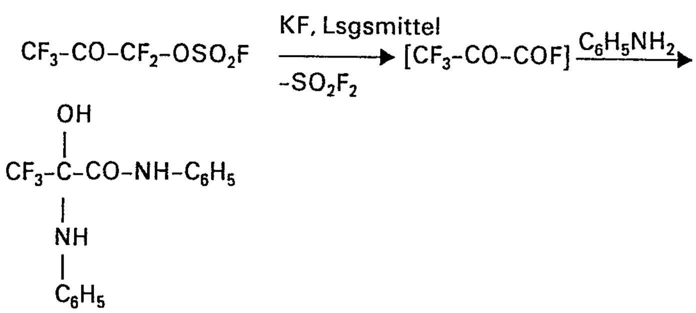


und in der US-PS 3 549 711 für ein sekundäres Fluorosulfat (mit equimolaren Mengen an KF) beschrieben

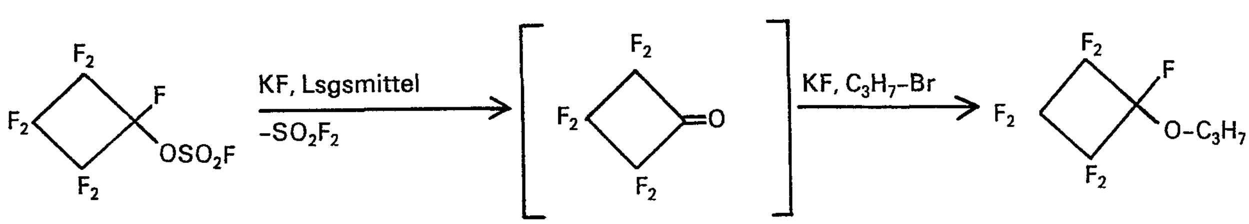

In diesen beiden Fällen wird aber die entstandene Perfluorcarbonylverbindung nicht isoliert, sondern direkt weiter umgesetzt, so dass ungeklärt bleibt, ob eine Isolierung der Perfluorcarbonylverbindungen unter diesen Bedingungen überhaupt möglich ist.

Aufgrund der vorstehend angeführten Literaturbeispiele für die Spaltung von primären und sekundären Fluorsulfaten war es nun überraschend, dass Fluorosulfate der Formel III mit katalytischen bis equimolaren Mengen an Alkalifluorid in Gegenwart oder Abwesenheit eines aprotisch polaren Lösungsmittels in Perfluorcarbonylverbindungen mit einer Sulfofluoridgruppe der allgemeinen Formel I überführt und die Produkte isoliert werden können.

Die als Katalysatoren für das erfindungsgemässe Verfahren eingesetzten Alkalifluoride (LiF, NaF, KF, RbF, CsF) können sowohl einzeln als auch in Abmischungen miteinander eingesetzt werden. Die Katalysatormenge liegt im allgemeinen zwischen etwa 1 und etwa 100 Mol %, bezogen auf die Ausgangsverbindung III (mit X = F). Wird ein Fluorosulfat der allgemeinen Formel III (mit X=Cl) eingesetzt, so muss zusätzlich die zu III equimolare Menge an Alkalifluorid eingesetzt werden, weil gleichzeitig die Sulfochloridgruppe in die Sulfofluoridgruppe umgewandelt wird.

Die Reaktion kann in Gegenwart oder Abwesenheit eines aprotischen polaren Lösungsmittels wie Acetonitril, Diglyme, Tetraglyme, Sulfolan, Dimethylsulfoxid usw. durchgeführt werden.

Die Reaktionstemperaturen liegen je nach dem verwendeten Katalysator und dem eventuell verwendeten Lösungsmittel zwischen −20 und +120°C.

Für die erfindungsgemässe Reaktion ist es praktisch ohne Bedeutung, in welcher Reihenfolge die Reaktionskomponenten und das eventuell verwendete Lösungsmittel zusammengegeben werden. Es ist allerdings von Vorteil, während der gesamten Dauer der Reaktion für eine gute Durchmischung des Ansatzes zu sorgen.

Nach einer bevorzugten Ausführungsart legt man das Alkalifluorid und das eventuell verwendete Lösungsmittel vor und tropft das Fluorosulfat III zu. Wenn nicht schon beim Zutropfen Reaktion eintritt, erhitzt man, bis Gasentwicklung auftritt. Nach beendeter Gasentwicklung wird der Ansatz destilliert.

Die nach dem erfindungsgemässen Verfahren hergestellten perfluorierten Carbonylverbindungen mit einer Sulfofluoridgruppe sind im allgemeinen farblose, feuchtigkeitsempfindliche Flüssigkeiten. Daher ist die Herstellung unter Ausschluss von Feuchtigkeit durchzuführen. Von den Verbindungen I sind diejenigen mit n = 2–7 neu (Formel I′ = Formel I mit n = 2–7).

Die Verbindungen I werden nach bekannten Methoden hauptsächlich zu perfluorierten Vinyl- oder Allylverbindungen mit noch einer Sulfofluoridgruppe im Molekül verarbeitet, die ihrerseits zu wertvollen Homo- und Copolymerisaten umgesetzt werden. Die Homo- und Copolymerisate werden z.B. (nach Verseifung der Sulfofluoridgruppen) als chemisch und thermisch widerstandsfähige Ionenaustauscher verwendet.

Der Fortschritt der Erfindung ergibt sich aus folgender vergleichender Betrachtung:

1) Herstellung von $FOC-CF_2-SO_2F$

Obwohl das erfindungsgemässe Verfahren zur Herstellung von $FOC-CF_2-SO_2F$ dem bekannten Verfahren [J. Am. Chem. Soc. 82, 6181 (1960)] hinsichtlich der Anzahl der Reaktionsschritte und der Gesamtausbeute unterlegen ist, stellt es trotzdem eine Verbesserung dar, weil der mit Explosionsgefahr verbundene Reaktionsschritt des bekannten Verfahrens vermieden wird.

2) Herstellung von $CF_3-CO-CF_2-SO_2F$

Das erfindungsgemässe Verfahren erlaubt erstmals die Isolierung von $CF_3-CO-CF_2-SO_2F$. Ausserdem ist die Vorstufe zur Herstellung dieser Verbindung in reinem Zustand auch in Glasgefässen lagerfähig. Die Gesamtausbeuten sind, ausgehend von Hexafluoraceton bzw. Hexafluorpropen, annähernd gleich. Es muss jedoch in Betracht gezogen werden, dass Hexafluoraceton aus Hexafluorpropen hergestellt werden muss. Wegen der zahlreichen veröffentlichten Verfahren sei hier nur auf die einstufigen Verfahren in DE-AS 2 624 349 und De-AS 2 738 010 hingewiesen. In günstigen Fällen wird dort bei Umsätzen von 10–15 Mol % eine Ausbeute von bis zu ~70% Hexafluoraceton erhalten.

Die folgende Gegenüberstellung soll den Weg – ausgehend von bekannten einfachen «Basisprodukten» – zu der Verbindung $CF_3-CO-CF_2-SO_2F$ nach dem Stand der Technik und nach der Erfindung illustrieren:

Bekannte Verfahren (Stand der Technik):

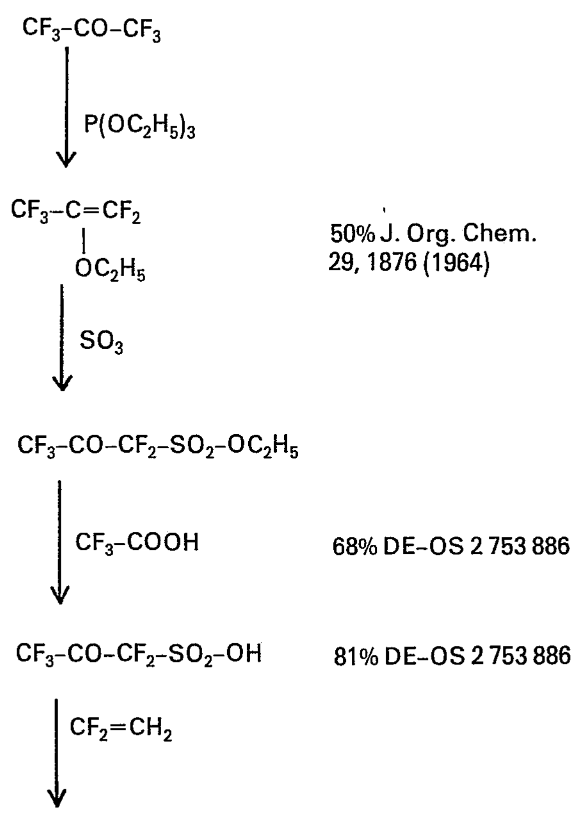

$CF_3-CO-CF_2-SO_2-O-CF_2-CH_3$

70% DE-OS 2 753 886

$CF_2=CF-CF_2-O-SO_2F$, KF

$CF_2-SO_2F$

|

$CF_3-CF-O-CF_2-CF=CF_2$ 34% DE-OS 2 753 886

Gesamtausbeute bis zur Vorstufe

$CF_3-CO-CF_2-SO_2-O-CF_2-CH_3$: 19%

Erfindung:

$CF_3-CF=CF_2$

$NaHSO_3$

J.Am.Chem.Soc. 75, 4595 (1953)

$CF_3-CHF-CF_2-SO_3H$ 64% (eigene Ausbeute 70%)

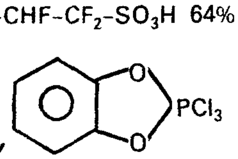

$CF_3-CHF-CF_2-SO_2Cl$ gleichzeitig eingereichte (und prioritätsgleiche) Anmeldung EP-A-47 946. 87%

KF, $KHF_2$

$CF_3-CHF-CF_2-SO_2F$ 61%

$e^-$ $HSO_3F$, $KSO_3F$ erfindungsgemäss

$OSO_2F$

|

$CF_3-CF-CF_2-SO_2F$ 46%

KF

$CF_3-CO-CF_2-SO_2F$ 46%

Gesamtausbeute bis zur Vorstufe

$OSO_2F$

|

$CF_3-CF-CF_2-SO_2F$: 17%

Die Erfindung wird nun durch die folgenden Beispiele näher erläutert:

Beispiel 1

Darstellung von 2-Fluorsulfato-perfluorethansulfonsäurefluorid ($F-SO_2-O-CF_2-CF_2-SO_2F$)

Die Elektrolysevorrichtung besteht aus einem mit einem äusseren Kühlmantel und einem Deckel versehenen zylindrischen Glasgefäss von 60 mm Innendurchmesser und etwa 100 mm Höhe. Die Zelle ist mit einem als Rückflusskühler wirkenden Trockeneiskühler, einem Gaseinleitungsrohr, einem Thermometer sowie den Stromzuführungen für die Elektroden versehen. Die Anode besteht aus einer Platte (100·20·3 mm) aus glasartigem Kohlenstoff, die am Deckel der Zelle befestigt ist und etwa 60 mm in den Elektrolyt eintaucht. Als Kathode dient ein 1,5 mm starker Platin-Draht, der in einem Abstand von etwa 20 mm parallel zur Anode angeordnet ist. Ein PTFE-ummantelter Magnetstab wird als Rührer benutzt. Als inerte Kühlflüssigkeit für den Kühlmantel verwendet man Perchlorethylen. Alle medienberührten Teile der Vorrichtung bestehen aus Glas, Platin oder PTFE.

Der Grundelektrolyt wird durch Zugabe von 12,5 g Kaliumchlorid zu 250 g destillierter Fluorsulfonsäure hergestellt. Dabei bildet sich Chlorwasserstoff, der durch Einleiten von trockenem Stickstoff aus der Lösung ausgetrieben wird. Anschliessend wird die Lösung etwa 4 Stunden bei 2A vorelektrolysiert.

Nach Zugabe von 120 g (0,65 Mol) 2-Hydroperfluorbutansulfonsäurefluorid wird bei einer Stromstärke von 3A und einer Zellspannung von 18 bis 22 V bis zu einem Ladungsdurchgang von 70 Ah elektrolysiert. Die Reaktionstemperatur beträgt 25–30°C.

Danach wird das Elektrolysegemisch im Scheidetrichter getrennt und die fluororganische Phase fraktioniert destilliert. Man erhält 110 g einer Fraktion Kp 87–90°C, die zu 95% aus 2-Fluorsulfato-perfluorethansulfonsäurefluorid besteht, das entspricht einer Ausbeute von etwa 54%, bezogen auf das eingesetzte Material.

$^{19}F$-NMR ($CDCl_3$)*): +52.7 (1F, $-O-SO_2-F$), +47.27 (1F, $-SO_2-F$), -81.45 (2F, $-CF_2-O$), -111.0 (2F, $-CF_2-S$).

*) Bei allen $^{19}F$-NMR-Spektren dient $CFCl_3$ als innerer Standard.

Beispiel 2

Darstellung von 2-Fluorsulfato-perfluorpropansulfonsäurefluorid ($CF_3-CF(OSO_2F)-CF_2-SO_2F$)

Unter Benutzung einer Elektrolysevorrichtung und nach Bereitung eines Grundelektrolyten, wie in Beispiel 1 beschrieben, werden 133 g (0.57 Mol) 2-Hydroperfluorpropansulfonsäurefluorid 45 Stunden bei 2A Stromstärke und 12–24 Volt Zellspannung elektrolysiert. Die Temperatur beträgt 30–35°C. Die fluororganische Phase wird durch Scheiden vom Elektrolyten abgetrennt und fraktioniert destilliert. Man erhält 123 g einer Fraktion, Kp 105°C, die gemäss dem $^{19}F$-NMR-Spektrum 70% 2-Fluorsulfato-perfluorpropansulfonsäurefluorid (neben 30% 1,2-Difluorsulfato-perfluorpropan) enthält.

$^{19}F$-NMR ($CDCl_3$): +53.09 (1F, O-$SO_2$-F), +47.8 (1F, -$SO_2$F), -76.14 (3F, -$CF_3$), -106.0 (2F, -$CF_2$-$SO_2$-), -135.3 (1F, > CF-O-).

Beispiel 3
Fluorsulfonyldifluoressigsäurefluorid
$F-SO_2-CF_2-COF$

Die Reaktion wird in einem Abzug durchgeführt.

In einem trockenen Kolben mit Magnetrührer, Thermometer, Tropftrichter, Rückflusskühler, der auf ca. -30°C gehalten wird, und nachgeschaltetem Blasenzähler legt man 3 g (0,02 Mol) Caesiumfluorid und 30ml Tetraethylenglykoldimethylether vor. Bei 15-20°C Innentemperatur tropft man 48 g (0,17 Mol) 2-Fluorsulfatoperfluorethansulfonsäurefluorid zu. Es tritt sofort Gasentwicklung ($SO_2F_2$) auf. Nachdem die Gasentwicklung beendet ist, wird der Ansatz auf 25-30°C erwärmt und ca. 30 Minuten bei dieser Temperatur gerührt. Die anschliessende Destillation liefert 18.8 g (61%) Fluorsulfonyldifluoressigsäurefluorid mit Kp 29-30°C (760 mm). Die spektralen Daten (IR und $^{19}F$-NMR-Spektrum) der Verbindung stimmen mit der angegebenen Struktur überein.

Beispiel 4
2-Oxoperfluorpropensulfonsäurefluorid
$CF_3-CO-CF_2-SO_2F$

Die Reaktion wird in einem Abzug durchgeführt.

In einem trockenen Kolben mit Magnetrührer, Tropftrichter, Thermometer, Rückflusskühler, Blasenzähler und nachgeschalteter Kältefalle (-78°C) legt man 8,7 g (0.15 Mol) Kaliumfluorid vor und tropft dann 50 g (~0.15 Mol) Substanzgemisch aus Beispiel 2 zu. Der Ansatz wird erwärmt. Bei ca. 95-100°C Innentemperatur setzt plötzlich eine heftige Reaktion ein. Es kondensiert viel Gas in der Kältefalle. Nachdem die Reaktion beendet ist, lässt man die Kältefalle auf RT aufwärmen. Die dabei zurückbleibende Flüssigkeit wird mit dem Kolbeninhalt destilliert. Es werden 13,2 g (46%, bezogen auf das im Ausgangsgemisch enthaltene $CF_3CF(OSO_2F)-CF_2-SO_2F$) 2-Oxoperfluorpropansulfonsäurefluorid mit Kp 49-50°C (755 mm) erhalten.

$^{19}F$-NMR ($CDCl_3$): +44.5 (1F, -$SO_2$F), -74.6 (3F, $CF_3$), -104.2 (2F, $CF_2$).

**Patentansprüche:**

1. Verfahren zur Herstellung von Perfluorcarbonyl-sulfonsäurefluoriden der Formel I

$$R_f-\underset{\underset{O}{\|}}{C}-(CF_2)_n-SO_2F \quad (I),$$

worin $R_f$ = F oder
Perfluoralkyl mit 1-10, vorzugsweise 1-8, insbesondere 1-3 C-Atomen, und
n = 0-7, bedeuten,
dadurch gekennzeichnet, dass man

a) Monohydroperfluoralkansulfonsäurehalogenide der Formel II

$$R_f-\underset{\underset{H}{|}}{CF}-(CF_2)_n-SO_2X \quad (II)$$

worin $R_f$ und n die gleiche Bedeutung wie in Formel I besitzen und X Cl oder F, vorzugsweise F bedeutet.

In einem Elektrolyten aus Fluorsulfonsäure und einem Alkalifluorsulfonat unter Verwendung von Anoden aus glasartigem Kohlenstoff

und von Kathoden aus einem üblichen, jedoch unter den Elektrolysebedingungen stabilen Material elektrolysiert, und

b) die dabei gebildeten Fluorsulfatoperfluoralkansulfonsäurehalogenide der Formel III

$$R_f-\underset{\underset{OSO_2F}{|}}{CF}-(CF_2)_n-SO_2X \quad (III)$$

worin $R_f$, X und n die gleiche Bedeutung wie in den Formeln I und II besitzen,

in Gegenwart von mindestens einem Alkalifluorid zu den Perfluorcabonyl-sulfonsäurehalogeniden der Formel I umsetzt.

2. Verfahren nach Anspruch 1, dadurch gekennzeichnet, dass man Stufe a) bei Temperaturen zwischen 0 und 100°C, vorzugsweise zwischen etwa 20 und etwa 40°C durchführt.

3. Verfahren nach den Ansprüchen 1-2, dadurch gekennzeichnet, dass man in Stufe b) pro Mol Fluorsulfatoperfluoralkansulfonsäurefluorid 0.1 bis 1 Mol Alkalifluorid verwendet.

4. Verfahren nach den Ansprüchen 1-2, dadurch gekennzeichnet, dass man in Stufe b) pro Mol Fluorsulfatoperfluoralkansulfonsäurechlorid 1.1 bis 2 Mol Alkalifluorid verwendet.

5. Verfahren nach den Ansprüchen 1-4, dadurch gekennzeichnet, dass man Stufe b) bei Temperaturen zwischen -20 und +120°C durchführt.

6. Fluorsulfatoperfluoralkansulfonsäurehalogenide der Formel III

$$R_f-\underset{\underset{OSO_2F}{|}}{CF}-(CF_2)_n-SO_2X \quad (III)$$

worin $R_f$ = F oder
Perfluoralkyl mit 1-10, vorzugsweise 1-8, insbesondere 1-3 C-Atomen,
X = Cl oder F, vorzugsweise F, und
n = 0-7 bedeuten.

**Claims**

1. A process for the preparation of perfluorocarbonylsulfonic acid fluorides of the formula I

$$R_f-\underset{\underset{O}{\|}}{C}-(CF_2)_n-SO_2F \quad (I),$$

in which $R_f$ denotes F or perfluoralkyl with 1-10, preferably 1-8 and in particular 1-3 C-atoms and n denotes a number from 0 to 7 characterized in

a) electrolyzing monohydroperfluoroalkanesulfonic acid halides of the formula II

$$R_f-\underset{\underset{H}{|}}{C}F-(CF_2)_n-SO_2X \quad (II)$$

in which $R_f$ and n have the same meaning as in formula I and X denotes Cl or F, preferably F, in an electrolyte comprising fluorosulfonic acid and an alkali metal fluorosulfonate, using anodes of glassy carbon and cathodes of a material which is customary, but stable under the electrolysis conditions, and

b) reacting the fluorosulfatoperfluoroalkanesulfonic acid halides thereby obtained, of the formula III

$$R_f-\underset{\underset{OSO_2F}{|}}{C}F-(CF_2)_n-SO_2X \quad (III)$$

in which $R_f$, X and n have the same meaning as in the formulae I and II, in the presence of at least one alkali metal fluoride, to give the perfluorocarbonyl sulfonic acid fluorides of the formula I.

2. A process as claimed in claim 1, characterized in carrying out stage

a) at temperatures between 0 and 100°C, preferably between 20 and 40°C.

3. A process as claimed in claims 1 and 2, characterized in using in stage

b) 0.1 to 1 mole of alkali metal fluoride per mole of fluorosulfatoperfluoroalkane-sulfonic acid fluoride.

4. A process as claimed in claims 1-2, characterized in using in stage b) 1.1 to 2 moles of alkali metal fluoride per mole of fluorosulfatoperfluoroalkane-sulfonic acid chloride.

5. A process as claimed in any of claims 1 to 4, characterized in carrying out stage b) at temperatures between -20 and +120°C.

6. Fluorosulfatoperfluoroalkanesulfonic acid halides of the formula III

$$R_f-\underset{\underset{OSO_2F}{|}}{C}F-(CF_2)_n-SO_2X \quad (III)$$

in which $R_f$ denotes F or perfluoroalkyl with 1-10, preferably 1-8 and in particular 1-3, C-atoms, X denotes Cl or F, preferably F,

and n denotes a number from 0 to 7.

**Revendications**

1. Procédé de préparation de fluorures d'acides perfluorocarbonyl-sulfoniques de formule I:

$$R_f-\underset{\underset{O}{\|}}{C}-(CF_2)_n-SO_2F \quad (I),$$

(dans laquelle $R_f$ représente F ou un groupe perfluoroalkyle comportant 1 à 10, avantageusement 1 à 8, en particulier 1 à 3, atomes de carbone, et n vaut 0 à 7), caractérisé en ce que:

a) l'on électrolyse des halogénures d'acides monohydrogénoperfluoroalcane-sulfoniques de formule II:

$$R_f-\underset{\underset{H}{|}}{C}F-(CF_2)_n-SO_2X \quad (II),$$

(dans laquelle $R_f$ et n ont le même sens que pour la formule I, et X représente Cl ou F, de préférence F) dans un électrolyte formé d'acide fluorosulfonique et d'un fluorosulfonate alcalin utilisant des anodes en carbone vitreux et des cathodes en un matériau usuel mais stable dans les conditions de l'électrolyse, et

b) on fait réagir, e, présence d'au moins un fluorure alcalin, les halogénures d'acides fluorosulfatoperfluoralcane-sulfoniques, ainsi formés, de formule III:

$$R_f-\underset{\underset{OSO_2F}{|}}{C}F-(CF_2)_n-SO_2X \quad (III),$$

(dans laquelle $R_f$, X et n ont le même sens que pour les formules I et II) pour obtenir les halogénures d'acides perfluorocarbonyl-sulfoniques de formule I.

2. Procédé selon la revendication 1, caractérisé en ce qu'on effectue l'étape a) à des températures comprises entre 0 et 100°C, avantageusement entre environ 20 et environ 40°C.

3. Procédé selon les revendications 1 ou 2, caractérisé en ce qu'on utilise dans l'étape b), par mole de fluorure de l'acide fluorosulfatoperfluoralcane-sulfonique 0,1 à 1 mole de fluorure alcalin.

4. Procédé selon l'une des revendications 1 et 2, caractérisé en ce qu'on utilise dans l'étape b), par mole de chlorure de l'acide flurorosulfatoperfluoralcane-sulfonique, 1,1 à 2 moles de fluorure alcalin.

5. Procédé selon l'une quelconque des revendications 1 à 4, caractérisé en ce qu'on effectue l'étape b) à des températures comprises entre -20°C et +120°C.

6. Halogénures d'acides fluorosulfatoperfluoralcane sulfoniques, caractérisés en ce qu'ils répondent à la formule III:

$$R_f-\underset{\underset{OSO_2F}{|}}{C}F-(CF_2)_n-SO_2X \quad (III)$$

dans laquelle $R_f$ représente F ou un groupe perfluoralkyle ayant 1 à 10, avantageusement 1 à 8, notamment 1 à 3, atomes de carbone,

X représente Cl ou F, avantageusement F, et n vaut 0 à 7.